# DEMANDE DE BREVET EUROPEEN

(11) **EP 1 407 733 A1**
(43) Date de publication de la demande: **14.04.2004**
(21) Numéro de dépôt: 03292292.4
(22) Date de dépôt: 18.09.2003
(51) Int. Cl.: A61F 9/013

(54) **TETE DE COUPE POUR MICROKERATOME**

(30) Priorité: 08.10.2002 FR 0212457
(71) Demandeur: Moria S.A., 92160 Antony (FR)
(72) Inventeur: Aufaure, Jean-Luc, 03210 Souvigny (FR); Frileux, David, 03210 Marigny (FR)
(74) Mandataire: Robert, Jean-Pierre

(57) **Abrégé**

Tête de coupe pour microkératome comportant un corps (2) et une lame de coupe (8) montée coulissante dans un logement traversant du corps le long d'une direction sensiblement parallèle à son fil de coupe (F), dans laquelle la lame (8) possède une fente (20) parallèle à son fil de coupe, ouverte sur l'un de ses bords latéraux (18), tandis que le logement possède une cloison (13) enfourchée par la lame lors de son introduction dans le corps et coopérant avec les bords de la fente (20) au guidage de la lame dans son mouvement parallèle à son fil coupant.

## Description

La présente invention concerne une tête de coupe pour microkératome.

### ARRIERE PLAN DE L'INVENTION

Un microkératome est un appareil de chirurgie ophtalmologique au moyen duquel on procède à des résections de cornée et, en particulier, à la découpe d'un volet cornéen afin de pratiquer dans le lit stromal découvert une ablation corrective. Cet appareil comporte un anneau de fixation sur l'oeil à opérer formant un guide linéaire rectiligne ou circulaire pour une tête de coupe. La tête de coupe possède un corps pourvu de moyens de guidage correspondant à ceux de l'anneau, à l'intérieur duquel une lame de coupe inclinée vers l'avant et vers l'anneau est animée d'un mouvement de va-et-vient le long de son fil de coupe. Le corps, en général, possède à l'avant de la lame un plateau d'aplanation de la cornée de sorte que le fil de coupe de la lame est situé en saillie sous le plateau, écarté de ce plateau d'une distance déterminée en fonction de l'épaisseur du volet cornéen à .réaliser.

Cette distance n'est en général pas réglable. Elle est déterminée par la position relative de la lame dans le corps de la tête de coupe. Or pour de multiples raisons, la position de cette lame est, dans les appareils connus, mal maîtrisée.

On rappellera que dans la plupart des kératomes connus, la tête de coupe comporte un logement transversal dans lequel la lame est guidée lors de son mouvement alternatif. Ce logement a une section dans un plan perpendiculaire au fil de coupe qui est sensiblement la même que celle de la lame, aux jeux fonctionnels près. On rappellera également que par lame on entend en ensemble formé par une plaque métallique dont le bord avant porte le fil coupant, à la manière d'une lame de rasoir, et par un élément en matière plastique en forme de talon rapporté sur cette lame pour saillir par rapport à l'une de ses faces et que l'on appelle navette. Cette navette en matière plastique présente au moins une face qui forme la face d'appui frottante de la lame sur une surface homologue de guidage du logement du corps. En outre, c'est dans cette navette en matière plastique qu'est ménagée une gorge dans laquelle coulisse un doigt excentrique du moteur engendrant le mouvement alternatif de la lame quand le corps de la tête de coupe est attelé au carter ou à l'enveloppe de ce moteur.

La navette est rapportée sur la plaque métallique par surmoulage, clipsage ou collage, techniques qui malgré tout le soin apporté à leur mise en oeuvre, entraînent une incertitude dans la précision de la position relative de la navette et de la plaque métallique proprement dite. Il s'ensuit que la distance de la face de glissement de la navette dans le logement du corps de la tête par rapport au fil de coupe peut varier d'une lame à l'autre et pour une lame, n'est pas connue avec précision suffisante pour qu'en service la position du fil coupant par rapport au plateau aplanateur soit bien maîtrisée. Cette imprécision influence de manière sensible, avec d'autres paramètres, l'épaisseur du capot cornéen. or, la tendance actuelle dans le domaine va vers la réalisation de capots cornéens de plus en plus minces.

### OBJET DE L'INVENTION

Par la présente invention on entend remédier au moins partiellement à cet inconvénient.

### BREVE DESCRIPTION DE L'INVENTION

La présente invention a pour objet une tête de coupe pour microkératome comportant un corps et une lame de coupe montée coulissante dans un logement traversant du corps le long d'une direction sensiblement parallèle à son fil de coupe, dans laquelle la lame possède une fente parallèle à son fil de coupe, ouverte sur l'un de ses bords latéraux, tandis que le logement possède une cloison enfourchée par la lame lors de son introduction dans le corps et coopérant avec les bords de la fente au guidage de la lame dans son mouvement parallèle à son fil coupant.

On assure par-là un guidage direct entre la tête et la lame permettant de s'affranchir des incertitudes de montage entre la lame et la navette d'entraînement de celle-ci par l'intermédiaire de laquelle, de manière connue, le guidage de la lame était assuré dans le logement de la tête.

Dans un mode préféré de réalisation, la cloison est de longueur inférieure à la longueur du logement prise dans la direction du coulissement, définissant ainsi une extrémité intérieure au logement qui forme butée de positionnement de la lame dans ce logement. Cette caractéristique permet d'une part d'éviter que la lame, à son introduction dans la tête, échappe au manipulateur et ressorte de l'autre côté du logement traversant et d'autre part, forme une butée pour une position déterminée de la lame transversalement dans le logement de la tête, ce qui aide à un accouplement rapide de la lame et de ses moyens d'entraînement à l'intérieur du corps de la tête.

Dans d'autres modes de réalisation, la cloison peut se réduire à un ou deux pions perpendiculaires au plan du logement de la lame dans lequel cette dernière va et vient, pions qui peuvent être métalliques dans une tête métallique ou plastique, rapportés de toute manière connue.

A l'effet précisément de pouvoir être entraîné par des moyens connus, la lame comporte une ouverture oblongue sensiblement perpendiculaire à la fente pour recevoir un doigt d'entraînement tournant de manière excentrée autour d'un axe orthogonal à cette fente. En outre, le logement possède sous la lame au droit de cette ouverture oblongue, un évidement autorisant la rotation du doigt d'entraînement qui traverse la lame.

Dans une variante de réalisation, il est prévu d'équiper de manière connue la lame sur l'une de ses faces d'un talon d'entraînement par lequel la lame coopère avec le doigt moteur. Dans ce cas, la partie de logement traversant le corps dans laquelle coulisse le talon de la lame sera d'un profil suffisamment dégagé pour qu'il n'y ait aucun contact entre le corps et le talon.

D'autres caractéristiques et avantages ressortiront de la description donnée ci-après à titre indicatif d'un mode de réalisation de l'invention et de ses variantes.

### BREVE DESCRIPTION DES DESSINS

Il sera fait référence aux dessins annexés parmi lesquels :
- la figure 1 est une vue extérieure d'une tête de kératome pivotant conforme à l'invention,
- la figure 2 est une vue en coupe de cette tête, selon le plan P de la figure 1, au niveau du logement traversant la tête,
- la figure 3 représente une première variante de réalisation de la lame selon l'invention,
- la figure 4 est une vue de côté schématique d'une variante de la tête de coupe selon l'invention,
- les figures 5 et 6 sont deux vues de face et en coupe d'une lame adaptée à la tête de la figure 4,
- la figure 7 est une vue schématique en coupe d'une variante de réalisation de l'invention.

### DESCRIPTION DETAILLEE DE L'INVENTION

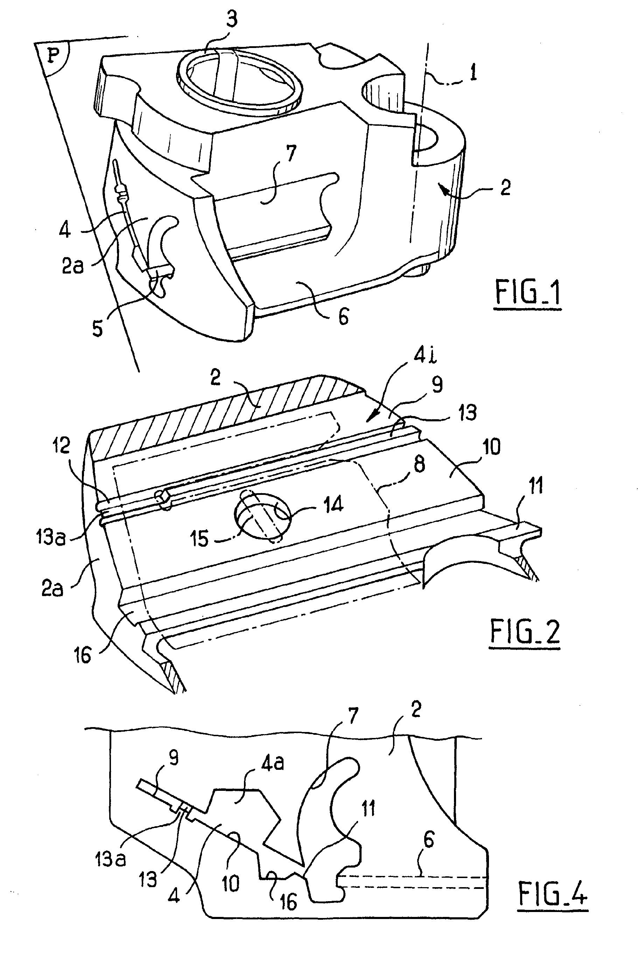

La tête représentée à la figure 1 est une tête de géométrie connue destinée à être adaptée à un microkératome motorisé pivotant autour d'un axe vertical 1 porté par l'anneau de fixation de l'oeil. Le corps 2 de cette tête peut être raccordé en 3 à un groupe moteur qui assure d'une part la rotation de la tête autour de l'axe 1 et d'autre part, le mouvement alternatif d'une lame placée dans un logement 4 du corps s'ouvrant en 5 à proximité du bord arrière d'un plateau aplanateur 6. Le volet cornéen découpé par la lame qui oscille dans la tête glisse sur un déflecteur courbe 7.

Les dispositions de l'invention sont mieux visibles à la figure 2 qui est une vue en coupe de la tête de la figure 1 par un plan P passant dans le logement 4 ménagé de manière traversante dans le corps 2 de la tête du microkératome. Cette figure 2 permet donc de découvrir la surface inférieure 4i du logement 4 sur laquelle repose la lame 8 représentée en trait mixte. Cette surface inférieure possède trois zones d'appui de la lame notées 9, 10, 11 depuis l'arrière de la tête jusqu'à l'extrémité du logement proche du plateau aplanateur 6. Ces surfaces 9, 10, 11 appartiennent à un même plan incliné. La surface 9 et la surface 10 sont séparées par une rainure 12 du fond de laquelle s'élève un cloisonnement 13 qui fait saillie au-dessus du plan commun des surfaces 9, 10, 11 et qui peut, le cas échéant, rejoindre la surface supérieure du logement 4. Ce cloisonnement 13 ne s'étend pas sur toute la longueur du logement 4 ; en effet, du côté 2a du corps de la tête de coupe qui est le côté d'introduction de la lame dàns le logement, ce cloisonnement possède une zone arasée 13a à un niveau inférieur au plan des surfaces 9, 10 et 11. Par ailleurs la surface 10 est pourvue d'un évidement 14 qui, situé sous la lame 8, permet l'évolution du doigt excentré créant le mouvement alternatif de la lame et appartenant à l'ensemble moteur connecté èn 3 à la tête de coupe. Le doigt excentré non représenté traverse la lame 8 par une lumière oblongue 15 qui est perpendiculaire au fil de coupe F. Enfin, une gorge 16 sépare la surface médiane 10 de la dernière surface 11 du logement 4, et constitue un réceptacle des éventuelles particules pouvant être produites lors du mouvement de la lame 8 dans la tête 2.

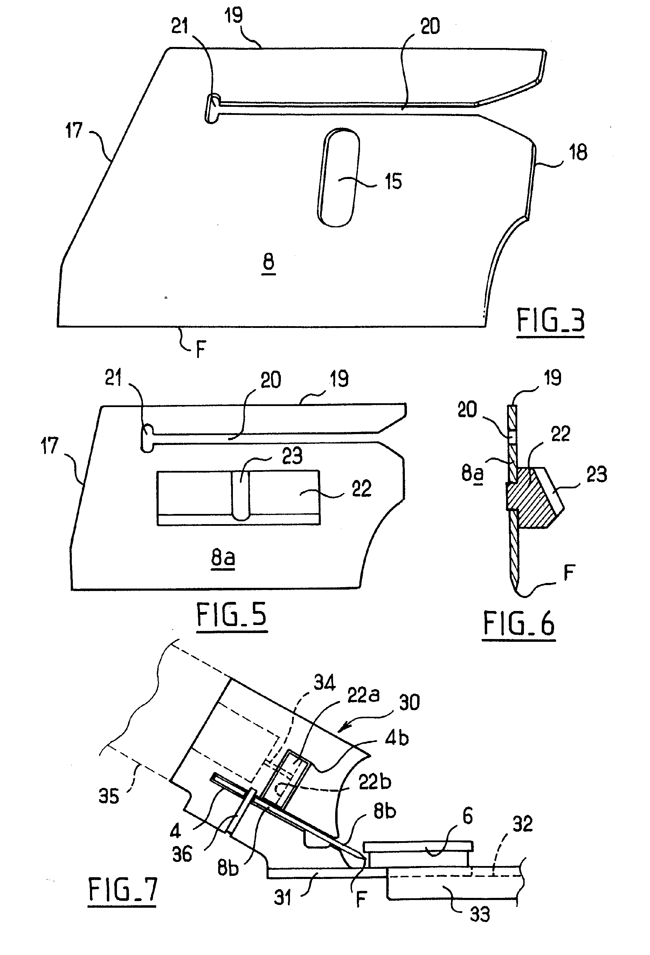

La lame 8 est représentée seule à la figure 3. Elle est formée par une plaque métallique mince avec un fil coupant F, deux bords latéraux 17 et 18 et un bord arrière 19. Elle comporte en partie supérieure, au voisinage du bord arrière 19, une fente 20 parallèle au bord coupant F débouchant d'une part sur le bord latéral 18 et d'autre part dans un évidement 21 prévu pour des raisons d'usinage et de confection de cette fente 20. La fente 20 s'évase en arrivant vers le bord 18, cet évasement permettant une introduction facile de la lame 8 dans le logement 4 par le côté 2a du corps 2 au moment où la fente 20 vient enfourcher le cloisonnement 13 au-delà de sa partie arasée 13a. Lorsque la lame est totalement enfoncée dans le logement 4, le cloisonnement 13 vient buter contre l'extrémité de l'évidement 21 de sorte que l'ouverture oblongue 15 de cette lame se trouve au-dessus de l'évidement 14, en un endroit qui est en intersection avec la trajectoire circulaire du doigt d'entraînement du groupe moteur lorsque ce dernier est relié à la tête de coupe 2. En effet, de manière connue, ce doigt d'entraînement est élastiquement escamotable le long de son axe afin qu'au montage de la tête sur le groupe moteur, il puisse se rétracter au contact de la surface supérieure de la lame 8 pour, à la première rotation, pénétrer à l'intérieur de l'ouverture oblongue 15.

A la figure 4 on a représenté une variante de réalisation de la tête de coupe, vue de profil, dans laquelle le logement 4 de la lame est surmonté d'un évidement 4a pour accommoder le talon ou navette 22 d'une variante de réalisation 8a de la lame représentée aux figures 5 et 6. On retrouve sur ces figures 4, 5 et 6 les éléments déjà décrits avec les mêmes références. On notera que de manière classique, c'est une encoche 23 de la navette 22 qui reçoit le doigt excentré d'entraînement de la lame. Le profil de la partie 4a du logement recevant la navette 22 est supérieur au profil de cette navette de manière qu'aucun contact entre la navette et le corps de la tête de coupe ne se produise.

La fente 20 des lames 8 et 8a peut être réalisée de manière extrêmement précise par rapport au fil de coupe. De la même manière on peut également réaliser le cloisonnement 13 à une distance bien maîtrisée du plateau aplanateur 6, si bien que lorsque l'on introduit la lame, le fil de coupe se trouve précisément situé par rapport au plateau aplanateur 6. On supprime ainsi l'incertitude de positionnement qui auparavant existait du fait du guidage de la lame dans la tête de coupe par l'intermédiaire de la navette. L'épaisseur du volet cornéen est ainsi mieux maîtrisée.

La coopération de la fente 20 et du cloisonnement 13 constitue enfin un détrompeur pour la mise en place de la lame dans la tête.

Alors que la description ci-dessus a été faite dans le cadre d'une tête de coupe pivotante, toutes les dispositions de l'invention peuvent être mises en oeuvre dans une tête de coupe à mouvement d'avance rectiligne perpendiculaire au fil de la lame au-dessus de l'anneau de fixation de l'oeil.

Alors que le guidage de la lame dans la tête a été décrit par coopération de la fente 20 et d'un cloisonnement 13, c'est-à-dire d'une saillie rectiligne continue sur au moins une certaine longueur, ce n'est pas sortir du cadre de l'invention que de prévoir, aux lieu et place du cloisonnement un, voire deux pions qui seraient chevauchés par le fente 20 de la lame 8, 8a. La figure 7 illustre schématiquement cette variante de réalisation dans une tête à avance rectiligne. La base du corps 30 de la tête de coupe porte deux queues d'aronde mâles 31 capables de coulisser dans des queues d'aronde femelles 32 ménagées dans un anneau 33 de fixation de l'oeil. La lame 8b possède une navette 22a logée dans un évidement 4b du logement 4 qui est sans contact avec la navette ou le talon 22a. Cette navette 22a est sensiblement perpendiculaire à la lame 8b et coopère par une encoche 22b avec un doigt d'entraînement tel que 34 d'un bloc moteur 35. Un pion 36 (ou de préférence deux pions 36 espacés) traversent le logement 4 de la lame 8b dans le corps 30 et sont chevauchés par la fente 20. Ils constituent, à l'instar de la cloison 13 les moyens de guidage de la lame dans la tête lors de son mouvement alternatif transversal.

## Revendications

1. Tête de coupe pour microkératome comportant un corps (2) et une lame de coupe (8) montée coulissante dans un logement (4) traversant du corps le long d'une direction sensiblement parallèle à son fil de coupe (F), **caractérisée en ce que** la lame (8) possède une fente (20) parallèle à son fil de coupe, ouverte sur l'un de ses bords latéraux (18), tandis que le logement possède une saillie (13, 36) enfourchée par la lame lors de son introduction dans le corps et coopérant avec les bords de la fente (20) au guidage de la lame dans son mouvement parallèle à son fil coupant.

2. Tête de coupe selon la revendication 1, **caractérisée en ce que** la saillie est une cloison (13) de longueur inférieure à la longueur du logement (4) prise dans la direction du coulissement, définissant ainsi une extrémité intérieure au logement qui forme butée de positionnement de la lame (8) dans le logement (4).

3. Tête de coupe selon la revendication 1, **caractérisée en ce que** la saillie est formée par au moins un pion (36) perpendiculaire au plan de la lame de coupe.

4. Tête de coupe selon la revendication 2 ou la revendication 3, **caractérisée en ce que** la lame (8) comporte une ouverture oblongue (15) sensiblement perpendiculaire à la fente (20) pour recevoir un doigt d'entraînement tournant de manière excentrée autour d'un axe orthogonal à la fente (20).

5. Tête de coupe selon la revendication 4, **caractérisée en ce que** le logement (4) possède sous la lame, au droit de l'ouverture oblongue (15), un évidement (14) autorisant la rotation du doigt d'entraînement.

6. Tête de coupe selon la revendication 2 ou la revendication 3, **caractérisée en ce que** la lame (8a, 8b) est équipée sur l'une de ses faces d'un talon (22, 22a) d'entraînement pourvu d'une encoche (22b, 23) orthogonale à la fente (20), destiné à recevoir un doigt d'entraînement tournant de manière excentrée autour d'un axe orthogonal à la fente (20).

7. Tête de coupe selon la revendication 6, **caractérisée en ce que** le profil de la partie (4a, 4b) du logement traversant du corps dans laquelle coulisse le talon (22, 22a) de la lame (8a, 8b) est sans contact avec ce talon.

8. Tête de coupe selon l'une quelconque des revendications précédentes, **caractérisée en ce que** le logement (4) possède au moins une gorge (16) parallèle à la cloison (13) et située entre elle et le fil de la lame, formant cavité de recueil de particules.

9. Lame de coupe pour microkératome comportant une plaque métallique avec un fil de coupe (F) et deux bords latéraux (17, 18) reliant les extrémités du fil de coupe (F) à un bord arrière (19), **caractérisée en ce qu'**elle comporte une fente (20) parallèle au fil de coupe (F) et débouchant sur l'un des bords latéraux (18) de la lame.

10. Lame selon la revendication 9, **caractérisée en ce que** le débouché de la fente (20) sur le bord (18) latéral est évasé.

11. Lame selon la revendication 9 ou la revendication 10, **caractérisée en ce qu'**elle comporte une ouverture oblongue (15) perpendiculaire au fil de coupe (F).

12. Lame selon la revendication 9, **caractérisée en ce qu'**elle comporte un talon (22, 22b) d'entraînement en saillie sur l'une de ses faces.
